# EUROPEAN PATENT SPECIFICATION

(11) **EP 3 860 607 B1**
(45) Date of publication and mention of the grant of the patent: **31.08.2022**
(21) Application number: 19794648.6
(22) Date of filing: 30.09.2019
(51) Int. Cl.: A61K 31/4745, A61P 25/18

(54) **COMPOUND (8AR,12AS,13AS)-5,8,8A,9,10,11,12,12A,13,13A- DECAIDRO-3-METHOXY-12-(ETHYLSULPHONYL)-6H- ISOCHINO [2,1-G] [1,6] NAFTIRIDINE FOR USE IN THE TREATMENT OF PSYCHOSES**
VERBINDUNG (8AR,12AS,13AS)-5,8,8A,9,10,11,12,12A,13,13A-DECAIDRO-3-METHOXY-12-(ETHYLSULPHONYL)-6H- ISOCHINO [2,1-G] [1,6] NAFTIRIDIN ZUR VERWENDUNG BEI DER BEHANDLUNG VON PSYCHOSEN
COMPOSÉ (8AR,12AS,13AS)-5,8,8A,9,10,11,12,12A,13,13A-DÉCAHYDRO-3-MÉTHOXY-12-(ÉTHYLSULFONYL)-6H-ISOQUINO [2,1-G] [1,6] NAPHTHYRIDINE POUR UNE UTILISATION DANS LE TRAITEMENT DE PSYCHOSES

(30) Priority: 01.10.2018 IT 201800009072
(43) Date of publication of application: 11.08.2021
(73) Proprietor: UNIVERSITA' DEGLI STUDI DI CAGLIARI, 09124 Cagliari (IT)
(72) Inventor: DEVOTO, Paola, 09124 Cagliari (IT); FRAU, Roberto, 09124 Cagliari (IT); GESSA, Gian Luigi, 09124 Cagliari (IT)
(74) Representative: Primiceri, Maria Vittoria
(86) International application number: PCT/IB2019/058285
(87) International publication number: WO 2020/070618

(56) References cited:
- BROSDA JAN ET AL: "[alpha]2-Adrenoceptors are targets for antipsychotic drugs", PSYCHOPHARMACOLOGY, SPRINGER VERLAG, BERLIN, DE, vol. 231, no. 5, 2 February 2014 (2014-02-02), pages 801-812, XP035369895, ISSN: 0033-3158, DOI: 10.1007/S00213-014-3459-8 [retrieved on 2014-02-02]
- VERONICA FAGERHOLM ET AL: "Autoradiographic characterization of [alpha]2C-adrenoceptors in the human striatum", SYNAPSE, vol. 62, no. 7, 1 July 2008 (2008-07-01), pages 508-515, XP055596399, US ISSN: 0887-4476, DOI: 10.1002/syn.20520

## Description

### Technical Field of the Invention

The present invention refers to the (8ar,12as,13aS)-5,8,8a,9,10,11,12,12a,13,13a-decahydro-3-methoxy-12-(ethylsulphonyl)-6H-isoquino[2,1-g][1,6]naphthyridine compound [CAS-186002-54-0], known in the literature under code RS-79948-197, for use in the treatment of schizophrenia. In particular, the present invention refers to the RS-79948-197 compound as an atypical antipsychotic for use in the treatment of schizophrenia.

In addition, the present invention refers to a pharmaceutical composition comprising the RS-79948-197 compound for use in the treatment of schizophrenia.

### Problem Underlying the Invention

According to the latest WHO estimates, schizophrenia affects about 0.3% of the world's population. Consequently, it is a relatively low prevalence disease. However, its costs, both economic and social, are very high, due to the early onset of the disease, low rates of remission and particularly severe disability affecting patients. In fact, people suffering from schizophrenia have high chances of being unemployed, homeless, of living in poverty, of having difficulty performing domestic and self-care activities and need continuous support from family members and available mental health services. The most onerous burden of schizophrenia is felt in the age group between 25 and 54 years, i.e., that in which individuals are usually more likely to be economically productive (Charlson et al., 2018). This results in significant economic deficits due to the productivity losses of individuals and their families, treatment costs and considerable expenses on health and social security systems. In Italy, for example, schizophrenia affects more than three hundred thousand people, with an economic expense exceeding EUR 3 billion, between direct and indirect costs. Spending on drugs amounts to about 10% of direct costs, but an effective therapy, however expensive, has a positive impact by lowering all other higher costs, in particular, represented by hospitalizations, loss of productivity and welfare costs charged on the families (Brugnoli et al., 2016).

### Description of the State of the Art

The pharmacological therapy of schizophrenia currently used in medical practice is based on the use of compounds having a so-called antipsychotic activity. Said compounds are subdivided into "*typical*"*,* or first-generation, and *"atypical",* or second-generation. The cost of antipsychotics varies from the minimum of old first-generation drugs (such as, for example, haloperidol, chlorpromazine), which however show severe side effects that affect adversely the therapeutic compliance with a consequent increase in expenditure, up to the highest cost of the most recent ones, often still patent-covered, for which the research investment has yet to be amortized. Since the spectrum of therapeutic effects of new antipsychotics has different features and nuances, with different prevalence of positive or negative effects among the drugs examined (McEvoy et al., 2006), the therapy must be adapted to the patient by varying the drug according to the symptomatic framework or adverse reactions manifested. For these reasons, the interest in new atypical antipsychotic drugs having more favourable features towards patients remains always high.

Currently, the prototype of atypical antipsychotics is clozapine, which, compared to traditional antipsychotics such as, for example, haloperidol, results also being effective on cognitive deficits and on positive symptoms, as well as on negative ones, without inducing hyperprolactinaemia and extrapyramidal disorders, which represent the most severe side effects of first-generation antipsychotics (Kane et al., 1988). The other atypical antipsychotics currently in use (such as olanzapine, quetiapine, risperidone, ziprasidone, aripiprazole, and the like) have more or less these features, but clozapine shows a better therapeutic profile, proving to be effective also in the forms of schizophrenia that are resistant to other drugs (Kane et al., 1988; Kinon and Lieberman, 1996; Meltzer and McGurk 1999; Chakos et al., 2001; McEvoy et al., 2006; Keefe et al., 2006; Leutch et al., 2009). Compared to typical antipsychotics, atypical antipsychotics generally show a spectrum of less severe side effects, with the notable exception of neutropenia which can be induced by clozapine. The most unpleasant side effects of second-generation (atypical) drugs are above all metabolic effects, with consequent weight gain, and, to a lesser extent, the most severe extrapyramidal effects or sedation induced by classical neuroleptics (Werner and Coveñas, 2014). The range of action of atypical antipsychotics has been attributed to their ability to bind, in addition to D2 receptors, also to other receptor classes, including, in particular, α₂-adrenergic receptors (Hertel et al., 1999; Hecht et al., 2012; Brosda et al., 2014). In particular, BROSDA JAN ET AL: "[alpha]2-Adrenoceptors are targets for antipsychotic drugs", PSYCHOPHARMACOLOGY,vol. 231, no. 5, 2 February 2014 (2014-02-02), pages 801-812, discloses the use of alpha-2 adrenergic antagonists alone or in combination with anti-psychotic drugs for the treatment of schizophrenia.

### Deficiencies in the State of the Art

In view of what noted above, it is clear that both typical and atypical antipsychotics have not yet solved satisfactorily the problem of providing a therapeutically effective and acceptable treatment for the treatment of schizophrenia. As already mentioned, also the most promising product, clozapine, is not immune to dangerous side effects such as for example, neutropenia.

Given the state of current knowledge and the drugs available to date, described above, the search for alternative antipsychotics is therefore always ongoing, not only to better understand the mechanisms and alterations underlying psychoses but, above all, to identify new effective therapeutic tools with less severe side effects.

### Technical Problem

There remains, therefore, a high demand from the medical community to have available a new effective, reliable product, safe for the patient, having at least the same (possibly better) advantageous features of the best atypical antipsychotics currently in use or under study.

In particular, the "goal" that is focused on is that of identifying a molecule having the advantageous features of clozapine but, possibly, without its side effects (such as, for example, the possible neutropenia) that prevent its use as a first-choice drug, thus allowing to intervene advantageously in earlier times with an adequate therapy that blocks or significantly slows down the progress of the disease and its symptoms, simultaneously improving the quality of life of the patient him/herself.

The object of the present invention is to provide an adequate solution to the technical problem set forth here above.

### Summary Description of the Invention

The Applicant has now unexpectedly found that the (8aR,12aS,13aS)-5,8,8a,9,10,11,12,12a,13,13a-decahydro-3-methoxy-12-(ethylsulphonyl)-6H-isoquino[2,1-g][1,6]naphthyridine compound [CAS-186002-54-0], commonly known in literature with the code RS-79948-197 (and with this indicated hereinafter, for convenience), is capable to give an adequate solution to the technical problem noted previously.

Therefore, an object of the present invention is the compound indicated with the code RS-79948-197 for the use in the treatment of schizophrenia, as reported in the appended independent claim.

Another object of the present invention is formed by a pharmaceutical composition comprising the aforementioned RS-79948-197 compound for the use in the treatment of schizophrenia, as reported in the appended independent claim.

Preferred embodiments of the present invention are reported in the appended dependent claims.

The preferred embodiments of the present invention reported in the following description, are reported therein by way of example only.

### Detailed Description of Invention

### Introduction

The compound of formula (I), commonly designed under code RS-79948-197, 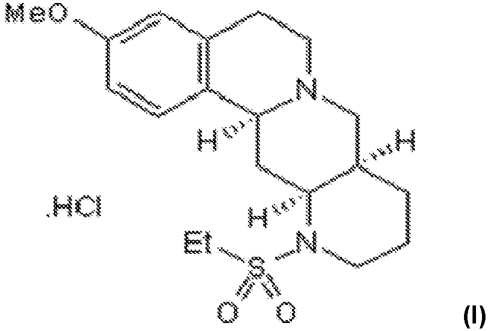 is known and marketed as a selective and potent α₂-adrenergic antagonist, (for example, it is available from the company Tocris, an authorized reseller of Roche Bioscience). All the present scientific literature, to the knowledge of the present inventors, agrees in defining and using this molecule as a selective α₂-adrenergic antagonist, based on binding studies (Milligan et al., 1997; Uhlen et al., 1998). However, during their studies, in addition to the expected anti-adrenergic effects, the present inventors have found other unexpected effects that cannot be explained by the activity of the selective α₂-adrenergic antagonist. Other types of tests were then carried out, aimed at verifying whether the unexpected effects of the drug were caused by its interaction with D2 receptors of dopamine and whether, possibly, it showed the features of an antipsychotic drug. Quite surprisingly, all the tests confirmed this hypothesis. Binding tests were conducted on human D2 dopaminergic receptors in transfected cells for D2 dopaminergic pharmacological activity in rabbit ear artery preparations, microdialysis experiments to assess the ability to influence brain dopamine levels in rats *in vivo*, as well as behavioural tests on rats about the antagonism of stereotypies induced by the selective D2 agonist quinpirol and on the deficit of "pre-pulse inhibition" induced by quinpirol and MK-801, an antagonist of NMDA receptors of glutamate. It should be noted that the latter test is considered the "gold standard" for the evaluation of the possible atypical antipsychotic activity of a drug.

### Brief Description of the Figures

Figure 1: shows the effect of pre-treatment with RS-79948-197 on amphetamine-induced hypermotility. The data are the average of 5 rats and are expressed in total counts. * P<0.05 vs. pre-treatment with physiological saline (Student's t-test).
Figure 2: shows the effect of treatment with RS 79948-197 on quinpirol-induced stereotypies. The data are the average of 6 rats and are expressed in total counts. * P<0.01 vs. treatment with physiological saline (Student's t-test).
Figure 3: shows the effect of treatment with RS-79948-197 or clozapine on PPI-deficit induced by MK-801. The data are the average of 4-8 rats. * P<0.05, ** P<0.01 vs. VEH+MK (one-way ANOVA followed by Tukey's Test). The groups pre-treated with RS-79948-197 or clozapine are not significantly different from the vehicle-treated group (ANOVA + Tukey's Test).
Figure 4: shows the effect of RS-79948-197 (at 3 mg/kg, i.p. administration, n = 3) and clozapine (at 2.5 mg/kg, i.p. administration, n = 3) on extracellular levels of dopamine (DA) measured by microdialysis in the rat medial prefrontal cortex (mPFC). The drugs were administered at time T=60 min, as indicated by the arrow. The values are expressed as a percentage of the basal value (mean +/-SEM).

### Description

In particular, the receptor binding study was conducted by analysing the ability to replace the binding of [³H]methyl-spiperone from the human D2_{long} receptor expressed by HEK-293 cells (Hall, D.A. and Strange, P.G., 1997, Brit. J. Pharmacol. 121:731-736). RS-79949-197 inhibited the binding with a Ki equal to 95 nM (i.e., with an affinity advantageously about twice that of clozapine).

In the test on the contractility of the rabbit ear artery (Steinsland, O.S. and Hieble, J.P., 1978, Science, 199:443-445), RS-79948-197 at the concentration of 100 nM antagonized by 70% the effect of the selective D2 agonist quinpirol, showing a power greater than that of sulpiride, a selective D2 antagonist, which at the same concentration inhibits by about 40%, while at higher concentrations RS-79948-197 has completely cancelled the quinpirol-induced release, resulting in an increase in the contractility of the preparation.

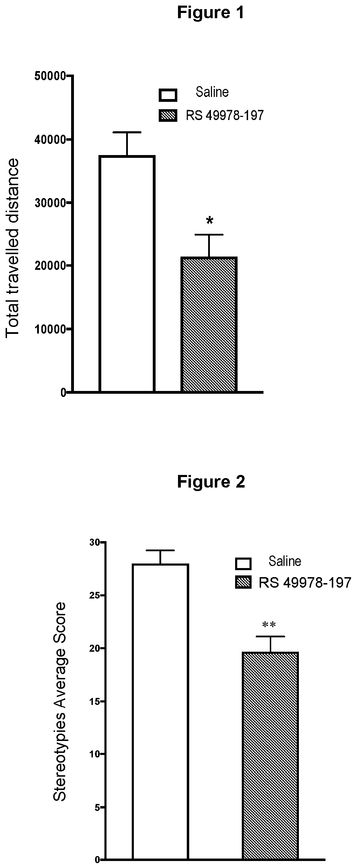

Some behavioural tests were also conducted to identify possible antipsychotic properties of RS-79948-197. In the amphetamine-induced hypermotility test (at 2.5 mg/kg, i.p. administration), considered predictive of efficacy on positive psychoses symptoms, RS-79948-197 (at 3 mg/kg, i.p. administration) significantly decreased the total distance travelled by rats in 120 minutes (see Figure 1 appended).

In turn, the appended Figure 2 shows that RS-79948-197 (at 3 mg/kg, i.p. administration) has also effectively reduced the stereotypies induced by the selective D2 agonist quinpirol (at 0.5 mg/kg, i.p. administration).

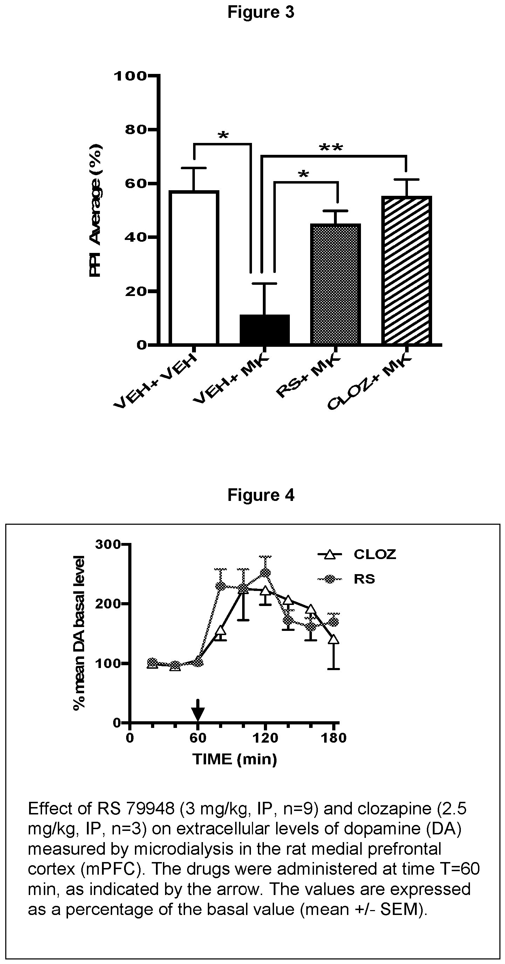

The activity of RS-79948-197 was also evaluated on the deficit of "pre-pulse inhibition" (PPI) induced by MK-801, an antagonist of NMDA receptors of glutamate. As already mentioned previously, this latter test is considered the "gold standard" for the evaluation of the possible atypical antipsychotic activity of a drug and is predictive of the efficacy on negative symptoms, as well as on positive ones, of schizophrenia. The test showed that the PPI deficit induced by MK-801 (at 0.1 mg/kg, i.p. administration) was effectively reduced by RS-79948-197 (at 3 mg/kg, i.p. administration), which brought the PPI values at the level of those of control animals (see appended Figure 3), in a manner comparable to the effect determined by clozapine (at a nearly double dose, i.e. at 5 mg/kg, i.p. administration).

Also, the effect of RS-79948-197 on the extracellular levels of dopamine in the rat prefrontal cerebral cortex, through microdialysis in free-moving rats, was further evaluated. As can be clearly seen in the appended Figure 4, the effects of the two drugs are substantially overlapping, as both have increased the extracellular concentration of dopamine up to about 250% of the respective basal value. Given the hypothesis of cortical hypodopaminergy in the etiopathogenesis of schizophrenia, the ability of a drug to increase the cortical dopamine is considered a favourable effect for its antipsychotic properties.

All these results considered globally have led to the conclusion that RS-79948-197, in addition to its well-known features as a selective compound for α₂-adrenergic receptors, advantageously presents the pre-clinical features of an atypical antipsychotic drug, thus resulting as a very promising first choice potential atypical antipsychotic drug for use in the treatment of psychoses, particularly of schizophrenia.

In view of all what set forth above, the present invention is directed to the following points:
- the (8aR,12aS,13aS)-5,8,8a,9,10,11,12,12a,13,13a-decahydro-3-methoxy-12-(ethylsulphonyl)-6H-isoquino[2,1-g][1,6]naphthyridine compound [CAS-186002-54-0] of formula (I), commonly known as RS-79948-197, as well as its pharmaceutically acceptable salts for the use in the treatment of schizophrenia;
- a pharmaceutical composition comprising the above RS-79948-197 compound, or its pharmaceutically acceptable salts for the use in the treatment of schizophrenia;
- the pharmaceutical composition described previously, further comprising one or more pharmacologically active compounds having the features of an atypical antipsychotic, such as those mentioned previously, for the use in the treatment of schizophrenia;
- the pharmaceutical composition described above, in which the aforementioned atypical antipsychotic compound is preferably clozapine, for the use in the treatment of schizophrenia;
- the above pharmaceutical composition, in which the reciprocal molar ratio between RS-79949-197 and said one or more atypical antipsychotic compounds is 99:1 to 1:99, for use in the treatment of schizophrenia; preferably, said molar ratio is 90:10 to 10:90; more preferably, said molar ratio is 80:20 to 20:80; more preferably, said molar ratio is 70:30 to 30:70; more preferably, said molar ratio is 60:40 to 40:60; even more preferably, said molar ratio is 55:45 to 45:55; even more preferably, said molar ratio is about 50:50; for example, said molar ratio is 50:50;
- the above pharmaceutical composition, further comprising one or more excipients, fillers, carriers, adjuvants, thickening agents, disintegrating agents, emulsifying agents, preservatives, known and commonly used in the formulative pharmaceutical technique, for use in the treatment of schizophrenia;
- the above pharmaceutical composition, in the form of a controlled-release formulation, for the use in the treatment of schizophrenia;
- the above pharmaceutical composition, in the form of an immediate-release formulation, or a sustained-release formulation, or a chronoactive-release formulation, for the use in the treatment of schizophrenia; the term chronoactive means that the active principle(s) is(are) treated differently (for example, encapsulated in suitable different chronoactive polymers), so as to release the active principle(s) in different time periods or at different positions of the intestinal tract;
- the above pharmaceutical composition, in the form of a formulation for oral administration or for injectable administration, for use in the treatment of schizophrenia.

The various pharmaceutical forms of the present invention for the use in the treatment of schizophrenia are prepared using preparation procedures and related apparatuses commonly known and used in the preparative pharmaceutical technique; consequently, the person skilled in the art will not have any difficulty in adopting the most suitable procedure and apparatus for realizing the desired pharmaceutical form according to the type of administration and dosage selected. Therefore, no further explanatory details are required in this regard.

Also as regards the dosage of the drug(s) of the present invention, the expert technician may select the same by making use of the information and teachings known for the use of the drugs already used in the field for this purpose. A preferred therapeutic dosage range for the RS-79948-197 compound is typically 0.03 mg to 10 mg/kg/day, depending on the patient's condition; preferably, 0.04 mg to 7 mg/kg/day; more preferably, 0.05 mg to 5 mg/kg/day.

From the results of the experiments of the present inventors, it was, therefore, possible to conclude that the RS-79948-197 compound presents in an advantageous and promising way the pre-clinical pharmacological features of the substances defined as "atypical antipsychotics", for example, clozapine, olanzapine, quetiapine, risperidone, ziprasidone, aripiprazole, and the like. Actually, with these drugs (in some respects better), it proved to be able to: a) bind both to dopamine D2 receptors and α₂-noradrenergic receptors; b) increase the extracellular levels of dopamine preferably in the prefrontal cortex; c) antagonize the stereotypies induced by the selective dopaminergic agonist quinpirol without inducing *per* se extrapyramidal effects; d) antagonize the destruction in rats of the pre-pulse inhibition induced by MK-801, a non-competitive antagonist of NMDA receptors; e) antagonize the amphetamine-induced hypermotility in rats.

These results are the result of pre-clinical research *in vitro* and *in vivo* on animals (rats) and, in the case of receptor binding analyses, also on cells transfected with human-type receptors.

### Industrial Applicability

In accordance with the invention, the results of the pre-clinical experiments conducted by the present authors indicate that the RS-79948-197 compound has the advantageous pharmacological features of the substances defined as "atypical antipsychotics", in particular clozapine. Actually, like these drugs, RS-79948-197 resulted able to: a) bind to both dopamine D2 receptors and a2-noradrenergic receptors; b) increase the extracellular levels of dopamine preferably in the prefrontal cortex; c) antagonize the stereotypies induced by the selective dopaminergic agonist quinpirol without inducing *per se* extrapyramidal effects; d) antagonize in the rat the destruction of the so-called pre-pulse inhibition induced by MK-801, a non-competitive antagonist of NMDA receptors; e) antagonize the amphetamine-induced hypermotility in the rat.

All this results extremely interesting and advantageous as regards the possible use of RS-79948-197 for the use in the treatment of schizophrenia.

## Claims

1. The (8aR,12aS,13aS)-5,8,8a,9,10,11,12,12a,13,13a-decahydro-3-methoxy-12-(ethylsulphonyl)-6H-isoquino[2,1-g][1,6]naphthyridine compound [CAS-186002-54-0], commonly known as RS-79948-197, or pharmaceutically acceptable salts thereof, for use in the treatment of schizophrenia.

2. A pharmaceutical composition comprising the RS-79948-197 compound of claim 1, for use in the treatment of schizophrenia.

3. The pharmaceutical composition for use according to claim 2, for use in the
treatment of schizophrenia, in which the therapeutic dosing of aforementioned RS-79948-197 compound is between 0.03 and 10 mg/kg/day.

4. The pharmaceutical composition for use according to claims 2-3, further
comprising one or more pharmaceutically active compounds having the features of an atypical antipsychotic, for use in the treatment of schizophrenia.

5. The pharmaceutical composition for use according to claim 4, in which said
preferred atypical antipsychotic compound is clozapine, for use in the treatment of schizophrenia.

6. The pharmaceutical composition for use according to claims 4-5, in which the
molar ratio between RS-79949-197 and said one or more atypical antipsychotic compounds is between 99:1 and 1:99, for use in the treatment of schizophrenia.

7. The pharmaceutical composition for use according to any one of claims 2 to 6,
further comprising one or more excipients, fillers, carriers, adjuvants, thickening agents, disintegrating agents, emulsifying agents, preservatives, which are known and commonly used in the formulative pharmaceutical technique, for use in the treatment of schizophrenia.

8. The pharmaceutical composition for use according to any one of claims 2 to 7,
in the form of a controlled-release formulation, for use in the treatment of schizophrenia.

9. The pharmaceutical composition for use according to claim 8, in the form of an
immediate-release formulation, or a sustained-release formulation, or a chronoactive-release formulation, for use in the treatment of schizophrenia.

10. The pharmaceutical composition for use according to any one of claims 2 to 9,
in the form of a formulation for oral administration or for injectable administration, for use in the treatment of schizophrenia.

## Patentansprüche

1. (8aR, 12aS, 13aS)-5,8,8a,9, 10, 11, 12, 12a, 13, 13a-Decahydro-3-Methoxy-12-(ethylsulfonyl)-6H-isochino[2,1-g][1,6]naphthyridin-[CAS-186002-54-0], allgemein bekannt als RS-79948-197, oder pharmazeutisch annehmbare Salze davon, zur Verwendung bei der Behandlung von Schizophrenie.

2. Pharmazeutische Zusammensetzung, umfassend die RS-79948-197-Verbindung nach Anspruch 1, zur Verwendung bei der Behandlung von Schizophrenie.

3. Pharmazeutische Zusammensetzung zur Verwendung nach Anspruch 2, zur Verwendung bei der Behandlung von Schizophrenie, wobei die therapeutische Dosierung der obengenannten RS-79948-197-Verbindung zwischen etwa 0,03 und etwa 10 mg/kg/Tag liegt.

4. Pharmazeutische Zusammensetzung zur Verwendung nach Ansprüche 2-3, ferner umfassend eine oder mehrere pharmazeutisch aktive Verbindungen mit atypischen antipsychotischen Merkmalen, zur Verwendung bei der Behandlung von Schizophrenie.

5. Pharmazeutische Zusammensetzung zur Verwendung nach Anspruch 4 , wobei die besagte bevorzugte atypische antipsychotische Verbindung Clozapin umfasst, zur Verwendung bei der Behandlung von Schizophrenie.

6. Pharmazeutische Zusammensetzung zur Verwendung nach Ansprüchen 4-5, wobei das Molverhältnis zwischen RS-79949-197 und der besagten einen oder mehreren atypischen antipsychotischen Verbindungen zwischen 99:1 und 1:99 liegt, zur Verwendung bei der Behandlung von Schizophrenie.

7. Pharmazeutische Zusammensetzung zur Verwendung nach einem der Ansprüche 2 bis 6, ferner umfassend einen oder mehrere Exzipienten, Füllstoffe, Träger, Adjuvantien, Verdickungsmittel, Sprengmittel oder Emulgatoren, Konservierungsmittel, die bekannt sind und normalerweise in der formulativen pharmazeutischen Technik verwendet werden, zur Verwendung bei der Behandlung von Schizophrenie.

8. Pharmazeutische Zusammensetzung zur Verwendung nach einem der Ansprüche 2 bis 7, in Form einer Formulierung mit kontrollierter Freisetzung, zur Verwendung bei der Behandlung von Schizophrenie.

9. Pharmazeutische Zusammensetzung zur Verwendung nach Anspruch 8, formuliert in Form einer Formulierung mit sofortiger Freisetzung oder einer Formulierung mit anhaltender Freisetzung oder einer Formulierung mit chronoaktiver Freisetzung, zur Verwendung bei der Behandlung von Schizophrenie.

10. Pharmazeutische Zusammensetzung zur Verwendung nach einem der Ansprüche 2 bis 9, in Form einer Formulierung zur oralen Verabreichung oder zur injizierbaren Verabreichung, zur Verwendung bei der Behandlung von Schizophrenie.

## Revendications

1. Composé [CAS-186002-54-0] (8aR, 12aS, 13aS)-5,8,8a,9,10, 11, 12, 12a, 13, 13a-décahydro-3- méthoxy-12-(éthylsulfonyl)-6H-isoquino[2,1-g][1,6]naphtyridine, couramment connu sous le nom de RS-79948-197, ou des sels pharmaceutiquement acceptables de celui-ci, pour utilisation dans le traitement de la schizophrénie.

2. Composition pharmaceutique comprenant le composé RS-79948-197 selon la revendication 1, pour utilisation dans le traitement de la schizophrénie.

3. Composition pharmaceutique pour utilisation selon la revendication 2 pour l'utilisation dans le traitement de la schizophrénie, dans lequel le dosage thérapeutique du composé RS-79948-197 susmentionné est compris entre environ 0,03 et environ 10 mg/kg/jour.

4. Composition pharmaceutique pour utilisation selon les revendications 2-3, comprenant en outre un ou plusieurs composés pharmaceutiquement actifs avec les propriétés d'un antipsychotique atypique pour utilisation dans le traitement de la schizophrénie.

5. Composition pharmaceutique pour utilisation selon la revendication 4, dans laquelle ledit composé antipsychotique atypique préféré est de la clozapine, pour utilisation dans le traitement de la schizophrénie.

6. Composition pharmaceutique pour utilisation selon les revendications 4 ou 5, dans laquelle le rapport molaire entre RS-79949-197 et lesdits un ou plusieurs composés antipsychotiques atypiques est compris entre 99:1 et 1:99, pour utilisation dans le traitement de la schizophrénie.

7. Composition pharmaceutique pour utilisation selon une quelconque des revendications 2 à 6, comprenant en outre un ou plusieurs excipients, charges, supports, adjuvants, des agents épaississants, désintégrants ou émulsifiants, conservateurs, connus et normalement utilisés dans la technique pharmaceutique formulative pour utilisation dans le traitement de la schizophrénie.

8. Composition pharmaceutique pour utilisation selon l'une quelconque des revendications 2 à 7, sous la forme d'une formulation à libération contrôlée, pour utilisation dans le traitement de la schizophrénie.

9. Composition pharmaceutique pour utilisation selon la revendication 8, sous forme d'une formulation à libération immédiate, ou d'une formulation à libération prolongée, ou d'une formulation à libération chrono-active, pour utilisation dans le traitement de la schizophrénie.

10. Composition pharmaceutique pour utilisation selon l'une quelconque des revendication 2 à 9, sous la forme d'une formulation pour administration orale ou pour administration injectable, pour utilisation dans le traitement de la schizophrénie.
